# EUROPEAN PATENT SPECIFICATION

(11) **EP 3 914 349 B1**
(45) Date of publication and mention of the grant of the patent: **01.03.2023**
(21) Application number: 20701443.2
(22) Date of filing: 20.01.2020
(51) Int. Cl.: A61P 1/02, A61P 17/00, A61P 31/04, A61P 31/10, A61P 31/12, A61K 6/00, A61K 9/00, A61K 33/00, A61K 47/02, A61K 47/10, A61K 47/36, A61K 47/38, A61K 9/06

(54) **COMPOSITION FOR THE TREATMENT OR PREVENTION OF INFECTIONS AND INFLAMMATIONS**
ZUSAMMENSETZUNG ZUR BEHANDLUNG ODER VORBEUGUNG VON INFEKTIONEN UND ENTZÜNDUNGEN
COMPOSITION POUR LE TRAITEMENT OU LA PRÉVENTION D'INFECTIONS ET D'INFLAMMATIONS

(30) Priority: 21.01.2019 DE 102019101434
(43) Date of publication of application: 01.12.2021
(73) Proprietor: Hager & Werken Gmbh & Co. KG, 47269 Duisburg (DE)
(72) Inventor: VAN DEN BOSCH, Willem Frederik, 03710 Calpe Provincia Alicante (ES)
(74) Representative: Nederlandsch Octrooibureau
(86) International application number: PCT/EP2020/051308
(87) International publication number: WO 2020/152115

(56) References cited:
- WO-A1-96/25916
- US-A1- 2008 274 206
- US-A1- 2015 056 272
- US-B1- 6 280 775

## Description

### FIELD OF THE INVENTION

This invention relates to compositions for use in the treatment or prevention of bacterial or fungal infections and inflammations (periodontitis and peri-implantitis) in the mouth. The invention further relates to compositions for use in the treatment or prevention of infections and inflammations of the skin or mucous membranes caused by HSV 1 and HSV 2 and bacterial superinfections.

### BACKGROUND OF THE INVENTION

Bacterial and fungal infections/inflammations in the mouth are common. Examples are periodontitis, peri-implantitis and bacterial infections/inflammations caused by injuries. Such bacterial infections and inflammations are commonly treated with antibiotics or disinfectants such as chlorohexidine. These compounds however also lead to disturbance of the healthy bacterial mouth-flora and/or lead to bacterial resistance.

It is an object of the invention to provide compositions that can be used to prevent or treat bacterial and fungal infections/inflammations in the mouth, particularly compositions that neither lead to disturbance of the healthy bacterial flora nor lead to bacterial resistance.

Herpes simplex viruses (HSV) have a worldwide distribution. Direct contact with or transmission through infected secretions is the most common mode of spreading. Virus particles can be transmitted by an infected person through kissing, hugging and touching. Virus particles can also be transmitted during the exercise of a profession such as in dentistry or hairdressing.

HSV 1 is transmitted by contact with oral secretions and HSV 2 by contact with genital secretions. About 40% of the world population has recurrent infections after having been infected with HSV 1. Up to 70% of the world population has recurrent infections after having been infected with HSV 2. In this respect, reference is made to Mandell, Douglas, and Bennett's Principles and Practice of Infectious Diseases, 8th Edition, 2015.

It is generally believed that most of the population get recurrent infections with HSV 1 or HSV 2 after they had a primary infection. Unclear is why and how the recurrent infections appear. It has however been demonstrated that HSV 1 and HSV 2 remain within trigeminal, sacral and vaginal ganglia between infections. Hence, even after recovery of a first infection/inflammation with HSV, there is direct risk of recurrent infections.

Typical diseases that can be related to HSV 1 and HSV 2 infections/inflammations are cold sores. Skin diseases such as eczema and psoriasis can also be related to herpetic infections/inflammations.

US2008/274206A1 discloses a stabilized, liquid, oxygen-releasing composition for use in the treatment of infections and lesions caused by the herpes simplex virus and psoriasis.

US2015/056272A1 discloses antimicrobial preparations for disinfection of articles or surfaces, antisepsis of skin or other body parts, prevention or minimization of scarring, and/or treatment or prophylaxis of dermal disorders. The antimicrobial preparations generally comprise from about 0.001 % to about 0.10 by weight of a metal chlorite in combination with from 0.001% to 0.05% of a peroxy compound.

WO96/25916A1 discloses a method for preparing a preparation for bleaching teeth or for treating skin complaints and mucous membrane disorders.

US6280775B1 discloses a liquid antimicrobial composition that is useful as a mouthwash for treating or reducing the risk of dental disease. The composition is prepared by mixing a first solution comprising a water soluble metal chlorite compound with a second solution comprising sodium persulfate and hydrogen peroxide.

It is an object of the invention to provide compositions that can be used to prevent or treat infections/inflammations caused by HSV 1 and HSV 2.

It is a further object of the invention to provide compositions that can be used to prevent or treat infections/inflammations that are caused by viruses having the same or a similar mechanism as HSV 1 and HSV 2.

It is a still further object of the invention to provide compositions that can be used for the purposes defined *supra* and that are further stable over time such that they can be used for said purposes for a prolonged period of time.

### SUMMARY OF THE INVENTION

The inventor has found that after being infected with HSV 1 or HSV 2 for the first time, virus particles remain at the facial skin, around the lips (HSV 1) or in the genital area (HSV 2). Recurrent infections with HSV 1 or HSV 2 occur when the remaining virus particles come into contact with damaged skin or damaged mucous membrane. Damages of the skin or mucous membranes can have many origins such as for example sunburn, dehydration due to dry air, mechanical damage, allergic reactions to cosmetics or food, and chemical damage due to acids, cleaning fluids, dishwasher salts or wash powders. These damages function as a port d'entrée for viruses.

Virus particles cannot move independently. Hence, virus particles come into contact with damaged skin or damaged mucous membrane by people replacing the virus particles themselves to the damaged area, *e.g.* by rubbing, scratching or by physical contact. When the virus enters a host cell via the damaged area, the virus infection may cause an inflammation reaction of the body.

The inventor has found that when:
a composition (I), which is obtainable by:
   preparing a mixture of:
      (a) glycerol;
      (b) one or more cellulose thickeners, preferably carboxymethyl cellulose; and
      (c) optionally one or more gums selected from xanthan gum, carrageenan, chicle gum (from sapodilla), guar gum, gum arabic, acacia gum, sodium alginate and tragacanth gum, and by adding to said mixture:
         (d) one or more cations C^{*t*+};
         (e) one or more anions derived from halogenoxides according to the general formula [XO*ᵥ*]⁻; and
         (f) water,
   with the proviso that no sorbitol is added,
   wherein C is a metal from group 1 or 2 of the periodic system, *t* = 1 or 2, X is a halogen atom and *v* = 1-4,
is mixed with a composition (II), which is obtainable by:
   preparing a mixture of:
      (g) glycerol;
      (h) one or more cellulose thickeners, preferably carboxymethyl cellulose; and
      (i) one or more gums selected from xanthan gum, carrageenan, chicle gum (from sapodilla), guar gum, gum arabic, acacia gum, sodium alginate and tragacanth gum, and by adding to said mixture:
         (j) one or more oxygen donors, wherein an oxygen donor consists of a cation C^{*t*+} and an anion according to the general formula [Z*_{y}*Oₓ]^{s-};and
         (k) water,
   with the proviso that no sorbitol is added,
   wherein C is a metal from group 1 or 2 of the periodic system, Z is chosen from the elements boron, manganese, carbon, sulfur, phosphorous, calcium and magnesium, and
   wherein O is the element oxygen, *y =* 0-4, *x* = 1-9, *s* = 1-6 and *t* = 1 or 2,
the combined compositions (I) + (II) result in a high yield of active oxygen, *i.e.* oxygen ions, which can be used to oxidize the envelope and the double-stranded DNA chain of the HSV virus such that the HSV virus is effectively oxidized and no further replication can take place. Hence, the combined compositions can also be used to prevent HSV infections or infections that act via the same mechanism, because any virus particles remaining after a first infection, can be effectively oxidized prior to possible contact with damaged skin or mucous membrane. In a similar way, the combined compositions have bactericidal and fungicidal activity through inactivation of anaerobic bacteria and fungi by oxidation. Hence, the combined compositions can be used to prevent or treat bacterial and fungal infections/inflammations in the mouth. Most of the anaerobic bacteria in the mouth, such as in plaque and in the pockets, are disappearing when treated with the composition as described herein in the form of for example a fluid, spray, toothpaste or gel. Moreover, the inventor has established that the active oxygen stimulates the underlying tissue to heal. It was further found that with this active-oxygen therapy, no bacterial resistance or disturbance of healthy bacteria flora occurred.

The compositions for use in the present invention do not comprise sorbitol. When adding sorbitol to a toothpaste, mouthwash or to similar products, using these products may result in a laxative effect which is not wanted. Moreover, sorbitol can react in the compositions for use in the present invention in a dehydration reaction from sorbitol to sorbitan thereby taking away active oxygen. Furthermore, as will become apparent from the appended examples, the inventor has established that sorbitol has a destabilizing effect on the combined compositions (I) + (II), resulting in the disappearance of a gel-like structure and the formation of a liquid phase. Therefore, the compositions are less efficacious when sorbitol is added.

The invention thus provides a kit of parts comprising compositions (I) and (II) in separate containers, wherein composition (I) is obtainable by:
preparing a mixture of:
   (a) glycerol;
   (b) one or more cellulose thickeners, preferably carboxymethyl cellulose; and
   (c) optionally one or more gums selected from xanthan gum, carrageenan, chicle gum (from sapodilla), guar gum, gum arabic, acacia gum, sodium alginate and tragacanth gum, and by adding to said mixture:
      (d) one or more cations C^{*t*+};
      (e) one or more anions derived from halogenoxides according to the general formula [XO*ᵥ*]⁻; and
      (f) water,

   with the proviso that no sorbitol is added,
   wherein C is a metal from group 1 or 2 of the periodic system, *t* = 1 or 2, X is a halogen atom and *v* = 1-4,
wherein composition (II) is obtainable by:
   preparing a mixture of:
      (g) glycerol;
      (h) one or more cellulose thickeners, preferably carboxymethyl cellulose; and
      (i) one or more gums selected from xanthan gum, carrageenan, chicle gum (from sapodilla), guar gum, gum arabic, acacia gum, sodium alginate and tragacanth gum, and by adding to said mixture:
         (j) one or more oxygen donors, wherein an oxygen donor comprises a cation C^{*t*+} and an anion according to the general formula [Z*_{y}*Oₓ]^{*s*-}; and
         (k) water,
      with the proviso that no sorbitol is added,
      wherein C is a metal from group 1 or 2 of the periodic system, Z is chosen from the elements boron, manganese, carbon, sulfur, phosphorous, calcium and magnesium, and
      wherein O is the element oxygen, *y =* 0-4, *x* = 1-9, *s* = 1-6 and *t* = 1 or 2,
for use in the treatment or prevention of bacterial or fungal infections and inflammations in the mouth, said treatment or prevention comprising mixing compositions (I) and (II) and administering the mixture of compositions to a subject, or
for use in the treatment or prevention of infections and inflammations of the skin or mucous membranes caused by HSV 1 and HSV 2, said treatment or prevention comprising mixing compositions (I) and (II) and administering the mixture of compositions to a subject.

The invention further provides a composition (III) obtainable by mixing compositions (I) and (II), wherein composition (I) is obtainable by:
preparing a mixture of:
   (a) glycerol;
   (b) one or more cellulose thickeners, preferably carboxymethyl cellulose; and
   (c) optionally one or more gums selected from xanthan gum, carrageenan, chicle gum (from sapodilla), guar gum, gum arabic, acacia gum, sodium alginate and tragacanth gum, and by adding to said mixture:
      (d) one or more cations C^{*t*+};
      (e) one or more anions derived from halogenoxides according to the general formula [XO*ᵥ*]⁻; and
      (f) water,
with the proviso that no sorbitol is added,
wherein C is a metal from group 1 or 2 of the periodic system, *t* = 1 or 2, X is a halogen atom and *v* = 1-4,
wherein composition (II) is obtainable by:
   preparing a mixture of:
      (g) glycerol;
      (h) one or more cellulose thickeners, preferably carboxymethyl cellulose; and
      (i) one or more gums selected from xanthan gum, carrageenan, chicle gum (from sapodilla), guar gum, gum arabic, acacia gum, sodium alginate and tragacanth gum, and by adding to said mixture:
         (j) one or more oxygen donors, wherein an oxygen donor comprises a cation C^{*t*+} and an anion according to the general formula [Z*_{y}*Oₓ]^{*s*-}; and
         (k) water,
   with the proviso that no sorbitol is added,
   wherein C is a metal from group 1 or 2 of the periodic system, Z is chosen from the elements boron, manganese, carbon, sulfur, phosphorous, calcium and magnesium, and
   wherein O is the element oxygen, *y =* 0-4, *x* = 1-9, *s* = 1-6 and *t* = 1 or 2,
for use in the treatment or prevention of bacterial or fungal infections and inflammations in the mouth, said treatment or prevention comprising administering composition (III) to a subject, or for use in the treatment or prevention of infections and inflammations of the skin or mucous membranes caused by HSV 1 and HSV 2, said treatment or prevention comprising administering composition (III) to a subject.

### DEFINITIONS

The term *'periodontitis',* as used herein, is considered to be synonymous with *'parodontitis'.*

The wording *'the composition is obtainable by reacting compounds*...' or similar wording, as used herein, means that the composition is directed to the product obtained or obtainable when the compounds are reacted. Hence, the wording *'the composition is obtainable by reacting compounds*...' and '*the composition is the reaction product of compounds*...' are considered interchangeable.

### DETAILED DESCRIPTION

In a first aspect, a kit of parts comprising compositions (I) and (II) in separate containers is provided, wherein composition (I) is obtainable by:
preparing a mixture of:
   (a) glycerol;
   (b) one or more cellulose thickeners, preferably carboxymethyl cellulose; and
   (c) optionally one or more gums selected from xanthan gum, carrageenan, chicle gum (from sapodilla), guar gum, gum arabic, acacia gum, sodium alginate and tragacanth gum, and by adding to said mixture:
      (d) one or more cations C^{*t*+};
      (e) one or more anions derived from halogenoxides according to the general formula [XO*ᵥ*]⁻; and
      (f) water,
with the proviso that no sorbitol is added,
wherein C is a metal from group 1 or 2 of the periodic system, *t* = 1 or 2, X is a halogen atom and *v* = 1-4,
wherein composition (II) is obtainable by:
preparing a mixture of:
   (g) glycerol;
   (h) one or more cellulose thickeners, preferably carboxymethyl cellulose; and
   (i) one or more gums selected from xanthan gum, carrageenan, chicle gum (from sapodilla), guar gum, gum arabic, acacia gum, sodium alginate and tragacanth gum, and by adding to said mixture:
      (j) one or more oxygen donors, wherein an oxygen donor comprises a cation C^{*t*+} and an anion according to the general formula [Z*_{y}*Oₓ]^{*s*-}; and
      (k) water,
   with the proviso that no sorbitol is added,
   wherein C is a metal from group 1 or 2 of the periodic system, Z is chosen from the elements boron, manganese, carbon, sulfur, phosphorous, calcium and magnesium, and wherein O is the element oxygen, *y =* 0-4, *x* = 1-9, *s* = 1-6 and *t* = 1 or 2,
for use in the treatment or prevention of bacterial or fungal infections and inflammations in the mouth, said treatment or prevention comprising mixing compositions (I) and (II) and administering the mixture of compositions to a subject.

In a second aspect, a composition (III) obtainable by mixing compositions (I) and (II) is provided, wherein composition (I) is obtainable by:
preparing a mixture of:
   (a) glycerol;
   (b) one or more cellulose thickeners, preferably carboxymethyl cellulose; and
   (c) optionally one or more gums selected from xanthan gum, carrageenan, chicle gum (from sapodilla), guar gum, gum arabic, acacia gum, sodium alginate and tragacanth gum, and by adding to said mixture:
      (d) one or more cations C^{*t*+};
      (e) one or more anions derived from halogenoxides according to the general formula [XO*ᵥ*]⁻; and
      (f) water,
with the proviso that no sorbitol is added,
wherein C is a metal from group 1 or 2 of the periodic system, *t* = 1 or 2, X is a halogen atom and *v* = 1-4,
wherein composition (II) is obtainable by:
preparing a mixture of:
   (g) glycerol;
   (h) one or more cellulose thickeners, preferably carboxymethyl cellulose; and
   (i) one or more gums selected from xanthan gum, carrageenan, chicle gum (from sapodilla), guar gum, gum arabic, acacia gum, sodium alginate and tragacanth gum, and by adding to said mixture:
      (j) one or more oxygen donors, wherein an oxygen donor comprises a cation C^{*t*+} and an anion according to the general formula [Z*_{y}*Oₓ]^{*s*-}; and
      (k) water,
   with the proviso that no sorbitol is added,
   wherein C is a metal from group 1 or 2 of the periodic system, Z is chosen from the elements boron, manganese, carbon, sulfur, phosphorous, calcium and magnesium, and wherein O is the element oxygen, *y =* 0-4, *x* = 1-9, *s* = 1-6 and *t* = 1 or 2,
for use in the treatment or prevention of bacterial or fungal infections and inflammations in the mouth, said treatment or prevention comprising administering composition (III) to a subject.

An example of fungal infections and inflammations that can be treated are infections and inflammations caused by Candida.

In a preferred embodiment, the bacterial or fungal infections and inflammations in the mouth are chosen from the group consisting of gingivitis, periodontitis and peri-implantitis.

In a third aspect of the invention, a kit of parts comprising compositions (I) and (II) in separate containers is provided, wherein composition (I) is obtainable by:
preparing a mixture of:
   (a) glycerol;
   (b) one or more cellulose thickeners, preferably carboxymethyl cellulose; and
   (c) optionally one or more gums selected from xanthan gum, carrageenan, chicle gum (from sapodilla), guar gum, gum arabic, acacia gum, sodium alginate and tragacanth gum, and by adding to said mixture:
      (d) one or more cations C^{*t*+};
      (e) one or more anions derived from halogenoxides according to the general formula [XO*ᵥ*]⁻; and
      (f) water,
with the proviso that no sorbitol is added,
wherein C is a metal from group 1 or 2 of the periodic system, *t* = 1 or 2, X is a halogen atom and *v* = 1-4,
wherein composition (II) is obtainable by:
preparing a mixture of:
   (g) glycerol;
   (h) one or more cellulose thickeners, preferably carboxymethyl cellulose; and
   (i) one or more gums selected from xanthan gum, carrageenan, chicle gum (from sapodilla), guar gum, gum arabic, acacia gum, sodium alginate and tragacanth gum, and by adding to said mixture:
      (j) one or more oxygen donors, wherein an oxygen donor comprises a cation C^{*t*+} and an anion according to the general formula [Z*_{y}*Oₓ]^{*s*-}; and
      (k) water,
   with the proviso that no sorbitol is added,
   wherein C is a metal from group 1 or 2 of the periodic system, Z is chosen from the elements boron, manganese, carbon, sulfur, phosphorous, calcium and magnesium, and
   wherein O is the element oxygen, *y =* 0-4, *x* = 1-9, *s* = 1-6 and *t* = 1 or 2,
for use in the treatment or prevention of infections and inflammations of the skin or mucous membranes caused by HSV 1, HSV 2 or viruses acting through the same mechanism, said treatment or prevention comprising mixing compositions (I) and (II) and administering the mixture of compositions to a subject.

In a fourth aspect of the invention, a composition (III) obtainable by mixing compositions (I) and (II) is provided, wherein composition (I) is obtainable by:
preparing a mixture of:
   (a) glycerol;
   (b) one or more cellulose thickeners, preferably carboxymethyl cellulose; and
   (c) optionally one or more gums selected from xanthan gum, carrageenan, chicle gum (from sapodilla), guar gum, gum arabic, acacia gum, sodium alginate and tragacanth gum, and by adding to said mixture:
      (d) one or more cations C^{*t*+};
      (e) one or more anions derived from halogenoxides according to the general formula [XO*ᵥ*]⁻; and
      (f) water,
with the proviso that no sorbitol is added,
wherein C is a metal from group 1 or 2 of the periodic system, *t* = 1 or 2, X is a halogen atom and *v* = 1-4,
wherein composition (II) is obtainable by:
preparing a mixture of:
   (g) glycerol;
   (h) one or more cellulose thickeners, preferably carboxymethyl cellulose; and
   (i) one or more gums selected from xanthan gum, carrageenan, chicle gum (from sapodilla), guar gum, gum arabic, acacia gum, sodium alginate and tragacanth gum, and by adding to said mixture:
      (j) one or more oxygen donors, wherein an oxygen donor comprises a cation C^{*t*+} and an anion according to the general formula [Z*_{y}*Oₓ]^{*s*-}; and
      (k) water,
      with the proviso that no sorbitol is added,
      wherein C is a metal from group 1 or 2 of the periodic system, Z is chosen from the elements boron, manganese, carbon, sulfur, phosphorous, calcium and magnesium, and
      wherein O is the element oxygen, *y =* 0-4, *x* = 1-9, *s* = 1-6 and *t* = 1 or 2,
for use in the treatment or prevention of infections and inflammations of the skin or mucous membranes caused by HSV 1, HSV 2 or viruses acting through the same mechanism, said treatment or prevention comprising administering composition(III) to a subject.

In a preferred embodiment, the infections and inflammations of the skin or mucous membranes caused by HSV 1 and HSV 2 are primary infections of the skin or mucous membranes which are followed in a lot of cases by secondary bacterial or fungal infections.

In another preferred embodiment, the infections and inflammations of the skin or mucous membranes related to HSV 1 and HSV 2 are chosen from eczema and psoriasis.

In still another preferred embodiment, the infection and inflammation of the skin or mucous membranes caused by viruses acting through the same mechanism as HSV 1 and HSV 2 is mouth ulcer.

Mixing of compositions (I) and (II) preferably takes place using a non-metal mixing device.

The metals C in the cations C^{*t*+} of compositions (I) and (II) are chosen independently. Both compositions can however contain the same metal C.

Preferred examples of cation C^{*t*+} are Na⁺, K⁺, Mg²⁺, Ca²⁺ and Mn²⁺.

The one or more anions derived from halogenoxides according to the general formula [XO*ᵥ*]⁻ in composition (I) are preferably chosen from the group consisting of perchlorate, chlorate, chlorite, hypochlorite, perioidate, iodate, iodite, hypoiodite, perbromate, bromate, bromite and hypobromite, more preferably the anion according to the general formula [XO*ᵥ*]⁻is hypochlorite.

As will be appreciated by the skilled person, the one or more cations C^{*t*+} and one or more anions [XO*ᵥ*]⁻ are added to composition (I) in the form of one or more salts C^{*t*+}[XO*ᵥ*]*ₜ*⁻. A very preferred salt that can be applied in composition (I) is sodium hypochlorite. Salts are preferably added to composition (I) as an aqueous solution.

The anion according to the general formula [Z*_{y}*Oₓ]^{*s*-} in the one or more oxygen donors of composition (II) is preferably chosen from the group consisting of perborate, metaborate, orthoborate, hypoborate, pyroborate, tetraborate, persulfate, peroxide, permanganate, percarbonate, perphosphate and hydrates thereof.

In a preferred embodiment, composition (II) comprises one oxygen donor, Z is boron and the anion according to the general formula [Z*_{y}*Oₓ]^{*s*-} in the oxygen donor is chosen from the group consisting of perborate, metaborate, orthoborate, hypoborate, pyroborate and tetraborate.

In another preferred embodiment, composition (II) comprises one oxygen donor, Z is boron and the anion according to the general formula [Z_{y}Oₓ]^{*s*-} is perborate tetrahydrate.

As will be appreciated by the skilled person, the cations C^{*t*+} and anions according to the general formula [Z*_{y}*Oₓ]^{*s*-} are added to composition (II) in the form of salts C^{*t*+}_{*s*/*t*}[Z*ᵥ*O*ₓ*]^{*s*-}, such as sodium percarbonate, magnesium peroxide, sodium percarbonate, potassium percarbonate, calcium percarbonate, sodium perborate trihydrate and sodium perborate tetrahydrate.

Very preferred salts that can be applied in composition (II) are sodium percarbonate, sodium perborate trihydrate and sodium perborate tetrahydrate.

In a very preferred embodiment, composition (II) comprises one oxygen donor, wherein the oxygen donor is added as the salt sodium perborate tetrahydrate.

In a very preferred embodiment, composition (II) comprises one oxygen donor, wherein Z is carbon and wherein the anion according to the general formula [Z*_{y}*Oₓ]^{*s*-} is percarbonate. Most preferably, composition (II) comprises one oxygen donor, wherein the oxygen donor is added as the salt sodium percarbonate. Salts are preferably added to composition (II) as an aqueous solution.

The inventor has found that it is essential that in the preparation of compositions (I) and (II), glycerol and the one or more cellulose thickeners, preferably carboxymethyl cellulose, are mixed first and that subsequently the remaining ingredients are added. Using this procedure sufficient active oxygen can be formed that is furthermore stabilized in the matrix of the formulation.

The inventor has established that adding the one or more gums selected from xanthan gum, carrageenan, chicle gum (from sapodilla), guar gum, gum arabic, acacia gum, sodium alginate and tragacanth gum has an unexpected advantageous effect on the stabilization of active oxygen in the mixture of combined compositions (I) and (II) or in composition (III). Accordingly, in an embodiment, the one or more gums selected from xanthan gum, carrageenan, chicle gum (from sapodilla), guar gum, gum arabic, acacia gum, sodium alginate and tragacanth gum is or are mandatory ingredients in composition (I) as defined herein. In a very preferred embodiment, the gum is xanthan gum. The one or more gums selected from xanthan gum, carrageenan, chicle gum (from sapodilla), guar gum, gum arabic, acacia gum, sodium alginate and tragacanth gum is or are mandatory ingredients in composition (II) as defined herein. The amount of the one or more gums selected from xanthan gum, carrageenan, chicle gum (from sapodilla), guar gum, gum arabic, acacia gum, sodium alginate and tragacanth gum in the combined compositions (I) and (II) or in composition (III) is preferably between 0.01 and 10 wt%, based on the total weight of combined compositions (I) and (II) or composition (III), preferably between 0.01 and 0.1 wt%, such as 0.025 wt%.

In another preferred embodiment, the mixture of compositions is allowed to rest and react for a period of at least 2 hours, more preferably for a period between 3 and 12 hours, even more preferably for a period between 6 and 8 hours, before the mixture is administered to a subject in need thereof.

In another preferred embodiment, the mixture of compositions (I) and (III) is allowed to rest and react for a period of at least 2 hours, more preferably for a period of between 3 and 12 hours, even more preferably for a period of between 6 and 8 hours, at a temperature between 4 and 20 °C, before the mixture of compositions (I) and (III) or before composition (III) is administered to a subject in need thereof.

If composition (III) or the mixture of compositions (I) and (II) is not directly administered after mixing compositions (I) and (II) or after allowing compositions (I) and (II) to react for the preferred periods indicated herein before, composition (III) or the mixture of compositions (I) and (II) is preferably kept at a temperature between 4 and 20 °C, for example in a refrigerator.

Without wishing to be bound by theory, it is hypothesized that the one or more anions derived from halogenoxides in composition (I) react with glycerol in the presence of water. The thickener of the cellulose type, preferably carboxymethyl cellulose, is used to slow down the reaction by increasing the viscosity of composition (I) and to keep active oxygen stabilized in the matrix of the formulation.

Without wishing to be bound by theory, it is hypothesized that the one or more oxygen donors in composition (II) react with glycerol.

When mixed, compositions (I) and (II) react with each other resulting in active oxygen, *i.e*. oxygen ions. This active oxygen causes oxidation of the envelope and the double-stranded DNA chain of HSV viruses such that the HSV virus is effectively oxidized and no further replication can take place. The combined compositions (composition (III) or the mixture of compositions (I) and (II)) can also be used to prevent HSV infections or infections that have the same mechanism, because any virus particles remaining after a first infection, can be effectively oxidized prior to possible contact with damaged skin or mucous membranes. In a similar way, the combined compositions have bactericidal and fungicidal activity through inactivation of anaerobic bacteria and fungi by oxidation. Hence, the combined compositions can be used to prevent or treat bacterial or fungal infections/inflammations in the mouth.

In another preferred embodiment, composition (III) or the mixture of compositions (I) and (II) as prepared or defined hereinbefore, is combined with one or more pharmaceutical acceptable carriers and/or pharmaceutical acceptable additives before composition (III) or the mixture of compositions (I) and (II) mixture is administered to a subject in need thereof. The use of pharmaceutical acceptable carriers may be advantageous in applying composition (III) or the mixture of compositions (I) and (II) to the inflammation in the oral cavity, onto the genital area or onto the skin. Preferred examples of pharmaceutical acceptable carriers and/or pharmaceutical acceptable additives are binders, thickeners, stabilizers, flavourings, perfumes and aromas, vitamins such as vitamin A and E, and co-enzymes such as Q10.

The one or more pharmaceutical acceptable carriers and/or pharmaceutical acceptable additives can however also be added to the compositions (I) and (II) before mixing them.

Examples of suitable stabilizers are sodium citrate, sodium fluoride, sodium hydroxide or citric acid.

Examples of suitable binders are glycerol and gums such as gum arabic and xanthan gum. A preferred binder is glycerol.

Additional thickeners may be added to increase the viscosity of the product. The amount needed depends on the desired viscosity of or the physical form of the composition to be administered. Examples of additional thickeners that can be applied are celluloses, or gums like xanthan gum or gum arabic.

Composition (III) or the mixture of composition (I) and composition (II), optionally combined with one or more pharmaceutical acceptable carriers and/or pharmaceutical acceptable additives as defined hereinbefore, can take the form of a liquid or a solid, particularly a powder, an aqueous solution, an aqueous dispersion, a gel, a mousse, a spray, a soap, a shampoo, a paste, a pocket-injection-gel an unguent or an ointment.

In an embodiment, composition (I) and composition (II) are mixed in amounts of 0.1 to 50 wt.% of composition (I) and 50 to 99.9 wt.% of composition (II), wherein the combined compositions (I) and (II) constitute 100 wt.%. In a preferred embodiment, composition (I) and composition (II) are mixed in amounts of 2 to 10 wt.% of composition (I) and 90 to 98 wt.% of composition (II), wherein the combined compositions (I) and (II) constitute 100 wt.%.

In an embodiment, the viscosity of the composition (III) or the mixture of composition (I) and composition (II), optionally combined with one or more pharmaceutical acceptable carriers and/or pharmaceutical acceptable additives as defined hereinbefore, is between 1 mPa·s and 500 Pa·s, wherein the viscosity is measured with a rheometer with a plate-plate geometry and a gap distance of 0.5 mm at a temperature of 25 °C.

In a particular embodiment, the treatment or prevention as defined hereinbefore comprises administering composition (III) or the mixture of composition (I) and composition (II), optionally combined with one or more pharmaceutical acceptable carriers and/or pharmaceutical acceptable additives as defined hereinbefore, by applying composition (III) or the mixture of composition (I) and composition (II) 1 to 8 times a day to the infected or inflamed areas.

In another preferred embodiment, the treatment or prevention of mouth ulcers as defined hereinbefore encompasses administering composition (III) or the mixture of composition (I) and composition (II), optionally combined with one or more pharmaceutical acceptable carriers and/or pharmaceutical acceptable additives as defined hereinbefore, by applying composition (III) or the mixture of composition (I) and composition (II) in the form of a mouthwash, a spray or an intraoral plaster to mouth ulcers in the oral area related to HSV, bacteria or fungi, or mixed infections of a first viral infection followed by a secondary bacterial of fungal infection.

In still another preferred embodiment the treatment or prevention of gingivitis, periodontitis or peri-implantitis as defined hereinbefore encompasses administering composition (III) or the mixture of composition (I) and composition (II), optionally combined with one or more pharmaceutical acceptable carriers and/or pharmaceutical acceptable additives as defined hereinbefore, by applying composition (III) or the mixture of composition (I) and composition (II) in the form of a toothgel or an anti-inflammative toothpaste to the oral area.

In still another preferred embodiment the treatment or prevention of fungal inflammations as defined hereinbefore encompasses administering composition (III) or the mixture of composition (I) and composition (II), optionally combined with one or more pharmaceutical acceptable carriers and/or pharmaceutical acceptable additives as defined hereinbefore, by applying composition (III) or the mixture of composition (I) and composition (II) in the form of a mouthwash to the oral area.

In a preferred embodiment, the composition (III) or the mixture of composition (I) and composition (II), preferably combined with one or more pharmaceutical acceptable carriers, is transparent. The composition can also be coloured.

### EXAMPLES

### Example 1: unguent for treating eczema or psoriasis

This example describes a method for preparing an unguent for treating eczema or psoriasis. In a first step, composition (I) was prepared. This composition was prepared by mixing the compounds listed below. Glycerol and carboxymethyl cellulose were mixed first and subsequently the remaining components were mixed in. The sodium hypochlorite was added to the composition as an aqueous solution. The glycerol contained a small amount of water. This has however been taken into account in the below composition such that 9 wt% glycerol amounts to 9 wt% of 100% pure glycerol. After mixing the compounds, the resulting mixture was allowed to rest and react for about 12 hours.

### Composition (I)

9 wt.% glycerol;
0.4 wt.% sodium hypochlorite;
75 wt.% water; and
0.3 wt.% carboxymethyl cellulose

Composition (II) was prepared directly after the mixing of the compounds of composition (I). Composition (II) was prepared by mixing the compounds listed below. Glycerol and carboxymethyl cellulose were mixed first and subsequently the remaining components were mixed in. The sodium percarbonate was added to the composition as an aqueous solution. The glycerol again contained a small amount of water. This has however been taken into account in the below composition such that 9 wt% glycerol amounts to 9 wt% of 100% pure glycerol. After mixing the compounds, the resulting mixture was allowed to rest and react for about 12 hours.

### Composition (II)

9 wt.% glycerol;
0.2 wt.% sodium percarbonate;
75.5 wt.% water; and
0.3 wt. % carboxymethyl cellulose.

Subsequently, composition (I) and composition (II) were combined and mixed. The resulting mixture was allowed to rest and react for about 12 hours.

In a further step, the following ingredients were added to the mixture of composition (I) and (II) (the amounts of the below ingredients are added twice such that composition (I) and the further ingredients result in 100 wt.% and such that composition (II) and the further ingredients result in 100 wt.%):

### Further ingredients

9 wt.% cetiol;
4 wt.% Dermofeel BGC (Evonik);
0.2 wt.% citric acid; and
2 wt.% Sepigel 305.

After thorough mixing with a non-metal mixing device for 120 minutes, the unguent for treating eczema or psoriasis was obtained.

### Example 2: treatment of atopic eczema and psoriasis

In a four-week application test, 20 patients with atopic eczema were treated with the unguent according to Example 1. Under clinical-dermatological circumstances, positive results were obtained. For 30% of the patients a healing of 100% was observed for 30% of the patients 50-80% healing, and for 40% of the patients 25-45% healing, wherein the healing percentages concern healing of the initially infected surface.

### Example 3: treatment of psoriasis

In a half year application test, 100 patients with psoriasis were treated with the unguent according to Example 1 and were observed. About 85% of the patients achieved good to excellent results, meaning that they had 80 to 100% less infected surface of the skin than before the treatment.

### Example 4: mouthwash

This example describes a method for preparing a mouthwash. The compositions (I) and (II) were prepared as described in Example 1 by mixing the compounds listed below.

### Composition (I)

10 wt.% glycerol;
0.6 wt.% sodium hypochlorite;
84.6 wt.% water; and
0.3 wt.% carboxymethyl cellulose.

### Composition (II)

10 wt.% glycerol;
0.6 wt.% sodium percarbonate;
84.6 wt.% water; and
0.3 wt.% carboxymethyl cellulose.

Subsequently, composition (I) and composition (II) were combined and mixed. The resulting mixture was allowed to rest and react for about 12 hours.

In a further step, the following ingredients were added to the mixture of composition (I) and (II) (the amounts of the below ingredients are added twice such that composition (I) and the further ingredients result in 100 wt.% and such that composition (II) and the further ingredients result in 100 wt.%):

### Further ingredients

2 wt.% castor oil;
2 wt.% sodium citrate;
0.1 wt.% citric acid; and
0.4 wt.% aroma RV 35641 peppermint.

After thorough mixing with a non-metal mixing device for 120 minutes, the mouthwash was obtained.

### Example 5: gel for the prevention of HSV 1 infections/inflammations

A gel was prepared analogous to the unguent of Example 1 and the mouthwash of Example 4. The overall composition of the ingredients used to prepare the gel was as follows:
17 wt.% glycerol;
5 wt.% silica;
2 wt.% sucrose;
3 wt.% Blanose 7LF;
1.5 wt.% sodium percarbonate;
1.5 wt.% sodium hypochlorite;
1 wt.% sodium gluconate;
1 wt% citric acid;
0.05 wt% aroma;
2 wt.% PEG-32; and
65.95 wt.% water.

Twenty people (patients) going into a situation in which they normally got HSV 1 infections/inflammations due to dry skin or excessive sunshine, such as during winter sport or summer vacation, were selected. These patients applied the gel 6 times a day to the infected/inflamed areas. Positive results were obtained for all twenty patients. These patients were able to prevent getting HSV 1 infections/inflammations on the treated areas during a longer period of time.

### Example 6: mouthwash for the treatment or prevention of fungal infections

The mouthwash of Example 4 was shown to inhibit the growth of a range of fungi, e.g. dermatophytes and Candida species when using the mouthwash 3-6 times a day.

### Example 7: toothgel

A toothgel was prepared analogous to the unguent of Example 1 and the mouthwash of Example 4. The overall composition of the ingredients used to prepare the toothgel was as follows:
64 wt.% glycerol;
0.4 wt.% magnesium sulfate;
0.045 wt.% sodium fluoride;
1 wt.% Blanose CMC 12M31XP;
10 wt.% Zeodent 113
0.4 wt.% sodium percarbonate;
0.4 wt.% sodium hypochlorite;
0.5 wt% citric acid;
1 wt% aroma RV35640;
2 wt.% PEG-1500; and
20.255 wt.% water.

### Example 8: toothgel for the treatment or prevention of periodontitis and peri-implantitis

Samples of bacteria from pockets of 28 patients who suffered from periodontitis were taken. Patients were treated with the toothgel of Example 7 comprising a composition of the invention. There was also a control group which got a placebo. Before treatment samples were taken of bacteria out of the pockets of the 28 patients, after 2 weeks of treatment using the toothpaste and after 4 weeks of treatment using the toothgel.

Fungi and anaerobic bacteria were dramatically lowered. The overall bacteria-flora in the mouth was not disturbed; a new balance existed.

In another experiment, a toothgel 10 times stronger (as regards the content of active oxygen) than the toothgel of Example 7 was punt into the pockets of 33 patients after cleaning, scaling, root planning or periodontal surgery. The gel was applied by a professional once or several times in one of more sessions. After application, the gel remained in the pockets for the rest of the day. No rinsing was performed. The patients were recommended to rinse with a mouthwash similar to that of Example 4 (but 4 times stronger as regards the content of active oxygen) starting the next day after treatment for about 1-4 weeks twice a day.

Most of the anaerobic bacteria were eliminated from the oral area and the pockets by using the toothgel. Also, several fungi were eliminated or brought to low numbers. The active oxygen in the toothgel was found to oxidise the biofilm in the pockets. The oxidising formula was able to penetrate deep into the pockets. These results were achieved within a short period of time (2-6 weeks).

After the treatment, it was recommended to use products with the oxidising formula, for instance the toothgel and/or the mouthwash, for a longer period to keep new anaerobic bacteria to a low level.

A new bacteria balance in the oral area was established. With this oxygen-therapy no bacterial resistance or disturbance of healthy bacteria flora occurred.

A compound such as CHX (chlorhexidine) is not selective in eliminating bacteria in the oral area. Therefore, this compound results in disturbance and misbalance of the bacteria flora as well as growth of fungi which can cause problems in the oral cavity. Moreover, CHX can only be used for a limited period of time and CHX does not work against fungal inflammation.

The use of antibiotics causes bacterial resistance in the long term. CHX and antibiotics do not work properly against periodontitis because the molecules of CHX and antibiotics are too large to penetrate into the biofilm of the pockets. So, both CHX and antibiotics are not suitable for treatment of periodontitis.

Peri-implantitis is caused by the same bacteria as periodontitis (parodontitis). The working mechanism for treating peri-implantitis and periodontitis is the same. After 3 months of treatment with the oxidising formula, 75% of the peri-implantitis cases were clinically cured. In 75% of these peri-implantitis cases re-osseo-integration was seen.

A microbiological study proved that the use of the mouthwash had a significant influence on the bacterial composition in the pockets without tooth brushing for 1 week, *i.e.* by just only rinsing the mouth with the mouthwash. *Actinobacillus actinomycetemcomitans, porphyromonas gingivalis* and *prevotella intermedia* were reduced to up to less than 5% of the original amount.

In case of extracting teeth it was very helpful to let patients rinse with a mouthwash according to the invention. For instance, in case of extracting wisdomteeth, rinsing with a mouthwash after and/or before treatment 3-6 times a day was found to be very helpful against infection and inflammation.

### Example 9: effect of xanthan gum on active oxygen

Mouthwashes in accordance with Example 4 were prepared, with xanthan gum (A) and without (B) xanthan gum. In the mouthwash with xanthan gum, the xanthan gum was added to only composition (II) in an amount of 0.025 wt.% based on the total weight of the combined compositions (I) and (II). Similarly, gels in accordance with Example 5 were prepared, with xanthan gum (C) and without (D) xanthan gum. In the gel with xanthan gum, the xanthan gum was added to only composition (II) in an amount of 0.025 wt.% based on the total weight of the combined compositions (I) and (II).

The presence of active oxygen in all formulations (A)-(D) was tested as a function of storage time at 20°C in a closed container, using H₂O₂ indicator strips. Results are given in Table 1.

**Table 1:**

| **Day** | **A.%O₂** | **B.%O₂** | **C.%O₂** | **D.%O₂** | **Rec.% A** | **Rec.% B** | **Rec.% C** | **Rec.% D** |
|---|---|---|---|---|---|---|---|---|
| 0 | 0.088 | 0.09 | 0.24 | 0.1 | 100 | 100 | 100 | 100 |
| 14 | 0.082 | 0.09 | 0.23 | 0.1 | 92 | 100 | 98 | 100 |
| 30 | 0.082 | 0.09 | 0.22 | 0.1 | 92 | 100 | 93 | 100 |
| 100 | 0.075 | 0.09 | 0.20 | 0.1 | 84 | 100 | 83 | 100 |
| 200 | 0.07 | 0.09 | 0.17 | 0.1 | 79 | 100 | 72 | 100 |

As can be inferred from Table 1, the addition of a gum such as xanthan gum has an unexpected advantageous effect on the stabilization of active oxygen in the mixture of combined compositions (I) and (II).

### Example 10: effect of sorbitol on stability and active oxygen

Mouthwashes in accordance with Example 4 were prepared, with sorbitol (A) and without (B) sorbitol. In the mouthwash with sorbitol, the sorbitol was added to composition (II) in an amount of 10 wt.% based on the total weight of the combined compositions (I) and (II). Similarly, gels in accordance with Example 5 were prepared, with sorbitol (C) and without (D) sorbitol. In the gel with sorbitol, the sorbitol was added to composition (II) in an amount of 10 wt.% based on the total weight of the combined compositions (I) and (II).

It was found that the gel-like structure of gel (C), with sorbitol, disappeared after 1 week storage at a temperature of 20°C and a liquid phase was formed. Gel (D), without sorbitol, on the other hand, remained stable for a long period of time. Similarly, the mouthwash (A), with sorbitol, became more fluid after 1 week, whereas mouthwash (B), without sorbitol, remained stable. It was further observed that addition of sorbitol in both the mouthwash and the gel resulted in an unwanted rapid decrease of the amount of active oxygen.

## Claims

1. Kit of parts comprising composition (I) and (II) in separate containers,
wherein composition (I) is obtainable by:
preparing a mixture of:
(a) glycerol;
(b) one or more cellulose thickeners, preferably carboxymethyl cellulose; and
(c) optionally one or more gums selected from xanthan gum, carrageenan, chicle gum (from sapodilla), guar gum, gum arabic, acacia gum, sodium alginate and tragacanth gum,
and by adding to said mixture:
(d) one or more cations C^{*t*+};
(e) one or more anions derived from halogenoxides according to the general formula [XO*ᵥ*]⁻; and
(f) water,
with the proviso that no sorbitol is added,
wherein C is a metal from group 1 or 2 of the periodic system, *t* = 1 or 2, X is a halogen atom and *v* = 1-4,
wherein composition (II) is obtainable by:
preparing a mixture of:
(g) glycerol; and
(h) one or more cellulose thickeners, preferably carboxymethyl cellulose; and
(i) one or more gums selected from xanthan gum, carrageenan, chicle gum (from sapodilla), guar gum, gum arabic, acacia gum, sodium alginate and tragacanth gum, preferably xanthan gum,
and by adding to said mixture:
(j) one or more oxygen donors, wherein an oxygen donor comprises a cation C^{*t*+} and an anion according to the general formula [Z*_{y}*O*ₓ*]^{*s*-}; and
(k) water,
with the proviso that no sorbitol is added,
wherein C is a metal from group 1 or 2 of the periodic system, Z is chosen from the elements boron, manganese, carbon, sulfur, phosphorous, calcium and magnesium, and wherein O is the element oxygen, *y* = 0-4, *x* = 1-9, *s* = 1-6 and *t* = 1 or 2,
for use in the treatment or prevention of bacterial or fungal infections and inflammations in the mouth, said treatment or prevention comprising mixing composition (I) and (II) and administering the mixture of compositions to a subject, wherein the bacterial or fungal infections and inflammations in the mouth are preferably chosen from the group consisting of gingivitis, parodontitis and peri-implantitis.

2. Composition (III) obtainable by mixing compositions (I) and (II),
wherein composition (I) is obtainable by:
preparing a mixture of:
(a) glycerol;
(b) one or more cellulose thickeners, preferably carboxymethyl cellulose; and
(c) optionally one or more gums selected from xanthan gum, carrageenan, chicle gum (from sapodilla), guar gum, gum arabic, acacia gum, sodium alginate and tragacanth gum,
and by adding to said mixture:
(d) one or more cations C^{*t*+};
(e) one or more anions derived from halogenoxides according to the general formula [XO*ᵥ*]⁻; and
(f) water,
with the proviso that no sorbitol is added,
wherein C is a metal from group 1 or 2 of the periodic system, *t* = 1 or 2, X is a halogen atom and *v* = 1-4,
wherein composition (II) is obtainable by:
preparing a mixture of:
(g) glycerol;
(h) one or more cellulose thickeners, preferably carboxymethyl cellulose; and
(i) one or more gums selected from xanthan gum, carrageenan, chicle gum (from sapodilla), guar gum, gum arabic, acacia gum, sodium alginate and tragacanth gum, preferably xanthan gum,
and by adding to said mixture:
(j) one or more oxygen donors, wherein an oxygen donor comprises a cation C^{*t*+} and an anion according to the general formula [*Z*_{y}O*ₓ*]^{s-}; and
(k) water,
with the proviso that no sorbitol is added,
wherein C is a metal from group 1 or 2 of the periodic system, Z is chosen from the elements boron, manganese, carbon, sulfur, phosphorous, calcium and magnesium, and wherein O is the element oxygen, *y* = 0-4, *x* = 1-9, *s* = 1-6 and *t* = 1 or 2,
for use in the treatment or prevention of bacterial or fungal infections and inflammations in the mouth, said treatment or prevention comprising administering composition (III) to a subject, wherein the bacterial or fungal infections and inflammations in the mouth are preferably chosen from the group consisting of gingivitis, parodontitis and periimplantitis.

3. Kit of parts comprising composition (I) and (II) in separate containers,
wherein composition (I) is obtainable by:
preparing a mixture of:
(a) glycerol;
(b) one or more cellulose thickeners, preferably carboxymethyl cellulose; and
(c) optionally one or more gums selected from xanthan gum, carrageenan, chicle gum (from sapodilla), guar gum, gum arabic, acacia gum, sodium alginate and tragacanth gum,
and by adding to said mixture:
(d) one or more cations C^{*t*+};
(e) one or more anions derived from halogenoxides according to the general formula [XO*ᵥ*]⁻; and
(f) water,
with the proviso that no sorbitol is added,
wherein C is a metal from group 1 or 2 of the periodic system, *t* = 1 or 2, X is a halogen atom and *v* = 1-4,
wherein composition (II) is obtainable by:
preparing a mixture of:
(g) glycerol;
(h) one or more cellulose thickeners, preferably carboxymethyl cellulose; and
(i) one or more gums selected from xanthan gum, carrageenan, chicle gum (from sapodilla), guar gum, gum arabic, acacia gum, sodium alginate and tragacanth gum, preferably xanthan gum,
and by adding to said mixture:
(j) one or more oxygen donors, wherein an oxygen donor comprises a cation C^{*t*+} and an anion according to the general formula [Z*_{y}*O*ₓ*]^{s-}; and
(k) water,
with the proviso that no sorbitol is added,
wherein C is a metal from group 1 or 2 of the periodic system, Z is chosen from the elements boron, manganese, carbon, sulfur, phosphorous, calcium and magnesium, and wherein O is the element oxygen, *y* = 0-4, *x* = 1-9, *s* = 1-6 and *t* = 1 or 2,
for use in the treatment or prevention of infections and inflammations of the skin or mucous membranes caused by HSV 1 and HSV 2, said treatment or prevention comprising mixing composition (I) and (II) and administering the mixture of compositions to a subject.

4. Composition (III) obtainable by mixing compositions (I) and (II),
wherein composition (I) is obtainable by:
preparing a mixture of:
(a) glycerol;
(b) one or more cellulose thickeners, preferably carboxymethyl cellulose; and
(c) optionally one or more gums selected from xanthan gum, carrageenan, chicle gum (from sapodilla), guar gum, gum arabic, acacia gum, sodium alginate and tragacanth gum,
and by adding to said mixture:
(d) one or more cations C^{*t*+};
(e) one or more anions derived from halogenoxides according to the general formula [XO*ᵥ*]⁻; and
(f) water,
with the proviso that no sorbitol is added,
wherein C is a metal from group 1 or 2 of the periodic system, *t* = 1 or 2, X is a halogen atom and *v* = 1-4,
wherein composition (II) is obtainable by:
preparing a mixture of:
(g) glycerol;
(h) one or more cellulose thickeners, preferably carboxymethyl cellulose; and
(i) one or more gums selected from xanthan gum, carrageenan, chicle gum (from sapodilla), guar gum, gum arabic, acacia gum, sodium alginate and tragacanth gum, preferably xanthan gum,
and by adding to said mixture:
(j) one or more oxygen donors, wherein an oxygen donor comprises a cation C^{*t*+} and an anion according to the general formula [Z*_{y}*O*ₓ*]^{s-}; and
(k) water,
with the proviso that no sorbitol is added,
wherein C is a metal from group 1 or 2 of the periodic system, Z is chosen from the elements boron, manganese, carbon, sulfur, phosphorous, calcium and magnesium, and wherein O is the element oxygen, *y* = 0-4, *x* = 1-9, *s* = 1-6 and *t* = 1 or 2,
for use in the treatment or prevention of infections and inflammations of the skin or mucous membranes caused by HSV 1 and HSV 2, said treatment or prevention comprising administering composition (III) to a subject.

5. Kit of parts for use according to claim 3 or composition (III) for use according to claim 4, wherein infections and inflammations of the skin or mucous membranes caused by HSV 1 and HSV 2 are primary infections of the skin or mucous membranes which are followed by secondary bacterial or fungal infections.

6. Kit of parts for use according to claim 3 or composition (III) for use according to claim 4, wherein infections and inflammations of the skin or mucous membranes related to HSV 1 and HSV 2 are chosen from the group consisting of eczema and psoriasis.

7. Kit of parts for use according to claim 3 or composition (III) for use according to claim 4, wherein the infection and inflammation of the skin or mucous membranes caused by viruses acting through the same mechanism as HSV 1 and HSV 2 is mouth ulcer.

8. Kit of parts or composition (III) for use according to any one of claims 1 to 7, wherein the one or more anions derived from halogenoxides according to the general formula [XOᵥ]⁻ in composition (I) are chosen from the group consisting of perchlorate, chlorate, chlorite, hypochlorite, perioidate, iodate, iodite, hypoiodite, perbromate, bromate, bromite and hypobromite, wherein:
(i) the one or more anions derived from halogenoxides according to the general formula [XOᵥ]⁻ in composition (I) preferably comprise hypochlorite; or
(ii) composition (I) preferably comprises one cation C^{*t*+} and one anion derived from halogenoxides according to the general formula [XOᵥ]⁻, said cation and anion being added to the composition as an aqueous solution of sodium hypochlorite.

9. Kit of parts or composition (III) for use according to any one of claims 1 to 8, wherein the anion according to the general formula [Z*ᵥ*Oₓ]^{s-} in the one or more oxygen donors of composition (II) are chosen from the group consisting of perborate, metaborate, orthoborate, hypoborate, pyroborate, tetraborate, persulfate, peroxide, permanganate, percarbonate, perphosphate, and hydrates thereof.

10. Kit of parts or composition (III) for use according to any one of claims 1 to 9, wherein Z is boron and the anion according to the general formula [Z*ᵥ*Oₓ]^{s-} in the one or more oxygen donors of composition (II) is chosen from the group consisting of perborate, metaborate, orthoborate, hypoborate, pyroborate, and tetraborate, and hydrates thereof, preferably perborate.

11. Kit of parts or composition (III) for use according to any one of claims 1 to 9, wherein Z is carbon and the anion according to the general formula [Z*ᵥ*Oₓ]^{s-} in the one or more oxygen donors of composition (II) is percarbonate.

12. Kit of parts or composition (III) for use according to any one of claims 1 to 11, wherein the mixture of compositions (I) and (II) or composition (III) is combined with one or more pharmaceutical acceptable carriers and/or pharmaceutical acceptable additives before the mixture of compositions (I) and (II) or composition (III) is administered to the subject in need thereof.

13. Kit of parts or composition (III) for use according to claim 12, wherein the one or more pharmaceutical acceptable carriers and/or pharmaceutical acceptable additives are chosen from the group consisting of binders, thickeners, stabilizers, flavourings, perfumes, aromas, vitamins and co-enzymes,
wherein preferably:
(i) the binder is chosen from the group consisting of glycerol and gums; or
(ii) the binder is chosen from the group consisting of glycerol and gums and the thickener is carboxymethylcellulose; or
(iii) the thickener is carboxymethylcellulose.

14. Kit of parts or composition (III) for use according to any one of claims 1 to 13, wherein the mixture of compositions (I) and (II) or wherein composition (III), optionally combined with one or more pharmaceutical acceptable carriers and/or pharmaceutical acceptable additives, takes the form of a liquid or a solid, particularly a powder, an aqueous solution, an aqueous dispersion, a gel, a mousse, a spray, a soap, a shampoo, a paste, a pocket-injection-gel an unguent or an ointment.

15. Kit of parts or composition (III) for use according to any one of claims 1 to 14, wherein the one or more gums selected from xanthan gum, carrageenan, chicle gum (from sapodilla), guar gum, gum arabic, acacia gum, sodium alginate and tragacanth gum, preferably xanthan gum, are mandatory ingredients in composition (I) and (II).

## Patentansprüche

1. Bausatz, der eine Zusammensetzung (I) und (II) in getrennten Behältern aufweist, wobei die Zusammensetzung (I) erhältlich ist mittels:
Herstellen eines Gemisches aus:
(a) Glycerol bzw. Glycerin;
(b) einem oder mehreren Cellulose-Verdickungsmitteln, vorzugsweise Carboxymethylcellulose; und
(c) optional einem oder mehreren Gummi bzw. Gummisorten, ausgewählt aus Xanthangummi, Carrageenan, Chiclegummi (aus Sapodilla), Guargummi, Gummi arabicum, Akaziengummi, Natriumalginat und Tragantgummi,
und mittels Zugeben von Folgendem zu diesem Gemisch:
(d) ein oder mehrerer Kationen C^{*t*+};
(e) ein oder mehrerer Anionen abgeleitet von Halogenoxiden der allgemeinen Formel [XO*ᵥ*]⁻; und
(f) Wasser,
und zwar mit der Maßgabe, dass kein Sorbitol zugesetzt wird, wobei:
C ein Metall der Gruppe 1 oder 2 des Periodensystems ist,
*t =* 1 oder 2 ist, X ein Halogenatom ist und *v* = 1-4 ist,
wobei die Zusammensetzung (II) erhältlich ist mittels:
Herstellen eines Gemisches aus:
(g) Glycerin; und
(h) einem oder mehreren Cellulose-Verdickungsmitteln, vorzugsweise Carboxymethylcellulose; und
(i) einem oder mehreren Gummi bzw. Gummisorten, ausgewählt aus Xanthangummi, Carrageen, Chiclegummi (aus Sapodilla), Guargummi, Gummi arabicum, Akaziengummi, Natriumalginat und Tragantgummi, vorzugsweise Xanthangummi,
und mittels Zugeben von Folgendem zu diesem Gemisch:
(j) eines oder mehrerer Sauerstoffdonatoren, wobei ein Sauerstoffdonator ein Kation C^{*t*+} und ein Anion gemäß der allgemeinen Formel [Z*_{y}*O*ₓ*]^{*s*-}aufweist; und
(k) Wasser,
und zwar mit der Maßgabe, dass kein Sorbitol zugesetzt wird, wobei:
C ein Metall der Gruppe 1 oder 2 des Periodensystems ist, Z aus den Elementen Bor, Mangan, Kohlenstoff, Schwefel, Phosphor, Calcium und Magnesium ausgewählt ist, und wobei O das Element Sauerstoff ist, *y* = 0-4, *x* = 1-9, *s* = 1-6 und *t* = 1 oder 2 ist,
zur Verwendung bei der Behandlung oder Vorbeugung von bakteriellen oder mykotischen Infektionen und Entzündungen im Mund, wobei die Behandlung oder Vorbeugung Mischen der Zusammensetzung (I) und (II) und Verabreichen des Gemisches der Zusammensetzungen an ein Subjekt aufweist, wobei die bakteriellen oder mykotischen Infektionen und Entzündungen im Mund vorzugsweise aus der Gruppe bestehend aus Gingivitis, Paradontitis und Periimplantitis ausgewählt sind.

2. Zusammensetzung (III), erhältlich durch Mischen der Zusammensetzungen (I) und (II), wobei die Zusammensetzung (I) erhältlich ist durch:
Herstellen eines Gemisches aus:
(a) Glycerol bzw. Glycerin;
(b) einem oder mehreren Cellulose-Verdickungsmitteln, vorzugsweise Carboxymethylcellulose; und
(c) optional einem oder mehreren Gummi bzw. Gummisorten, ausgewählt aus Xanthangummi, Carrageenan, Chiclegummi (aus Sapodilla), Guargummi, Gummi arabicum, Akaziengummi, Natriumalginat und Tragantgummi,
und mittels Zugeben von Folgendem zu diesem Gemisch:
(d) ein oder mehrerer Kationen C^{*t*+};
(e) ein oder mehrerer Anionen abgeleitet von Halogenoxiden der allgemeinen Formel [XO*ᵥ*]⁻; und
(f) Wasser,
und zwar mit der Maßgabe, dass kein Sorbitol zugesetzt wird, wobei:
C ein Metall der Gruppe 1 oder 2 des Periodensystems ist,
*t =* 1 oder 2 ist, X ein Halogenatom ist und *v* = 1-4 ist,
wobei die Zusammensetzung (II) erhältlich ist mittels:
Herstellen eines Gemisches aus:
(g) Glycerin; und
(h) einem oder mehreren Cellulose-Verdickungsmitteln, vorzugsweise Carboxymethylcellulose; und
(i) einem oder mehreren Gummi bzw. Gummisorten, ausgewählt aus Xanthangummi, Carrageen, Chiclegummi (aus Sapodilla), Guargummi, Gummi arabicum, Akaziengummi, Natriumalginat und Tragantgummi, vorzugsweise Xanthangummi,
und mittels Zugeben von Folgendem zu diesem Gemisch:
(j) eines oder mehrerer Sauerstoffdonatoren, wobei ein Sauerstoffdonator ein Kation C^{*t*+} und ein Anion gemäß der allgemeinen Formel [*Z*_{y}O*ₓ*]^{*s*-}aufweist; und
(k) Wasser,
und zwar mit der Maßgabe, dass kein Sorbitol zugesetzt wird, wobei:
C ein Metall der Gruppe 1 oder 2 des Periodensystems ist, Z aus den Elementen Bor, Mangan, Kohlenstoff, Schwefel, Phosphor, Calcium und Magnesium ausgewählt ist, und wobei O das Element Sauerstoff ist, *y* = 0-4, *x* = 1-9, *s* = 1-6 und *t* = 1 oder 2 ist,
zur Verwendung bei der Behandlung oder Vorbeugung von bakteriellen oder mykotischen Infektionen und Entzündungen im Mund, wobei die Behandlung oder Vorbeugung Verabreichen der Zusammensetzung (III) an ein Subjekt aufweist, wobei die bakteriellen oder mykotischen Infektionen und Entzündungen im Mund vorzugsweise aus der Gruppe bestehend aus Gingivitis, Paradontitis und Periimplantitis ausgewählt sind.

3. Bausatz, der eine Zusammensetzung (I) und (II) in getrennten Behältern aufweist, wobei die Zusammensetzung (I) erhältlich ist mittels:
Herstellen eines Gemisches aus:
(a) Glycerol bzw. Glycerin;
(b) einem oder mehreren Cellulose-Verdickungsmitteln, vorzugsweise Carboxymethylcellulose; und
(c) optional einem oder mehreren Gummi bzw. Gummisorten, ausgewählt aus Xanthangummi, Carrageenan, Chiclegummi (aus Sapodilla), Guargummi, Gummi arabicum, Akaziengummi, Natriumalginat und Tragantgummi,
und mittels Zugeben von Folgendem zu diesem Gemisch:
(d) ein oder mehrerer Kationen C^{*t*+};
(e) ein oder mehrerer Anionen abgeleitet von Halogenoxiden der allgemeinen Formel [XO*ᵥ*]⁻; und
(f) Wasser,
und zwar mit der Maßgabe, dass kein Sorbitol zugesetzt wird, wobei:
C ein Metall der Gruppe 1 oder 2 des Periodensystems ist,
*t =* 1 oder 2 ist, X ein Halogenatom ist und *v* = 1-4 ist,
wobei die Zusammensetzung (II) erhältlich ist mittels:
Herstellen eines Gemisches aus:
(g) Glycerin; und
(h) einem oder mehreren Cellulose-Verdickungsmitteln, vorzugsweise Carboxymethylcellulose; und
(i) einem oder mehreren Gummi bzw. Gummisorten, ausgewählt aus Xanthangummi, Carrageen, Chiclegummi (aus Sapodilla), Guargummi, Gummi arabicum, Akaziengummi, Natriumalginat und Tragantgummi, vorzugsweise Xanthangummi,
und mittels Zugeben von Folgendem zu diesem Gemisch:
(j) eines oder mehrerer Sauerstoffdonatoren, wobei ein Sauerstoffdonator ein Kation C^{*t*+} und ein Anion gemäß der allgemeinen Formel [Z*_{y}*O*ₓ*]^{*s*-}aufweist; und
(k) Wasser,
und zwar mit der Maßgabe, dass kein Sorbitol zugesetzt wird, wobei:
C ein Metall der Gruppe 1 oder 2 des Periodensystems ist, Z aus den Elementen Bor, Mangan, Kohlenstoff, Schwefel, Phosphor, Calcium und Magnesium ausgewählt ist, und wobei O das Element Sauerstoff ist, *y* = 0-4, *x* = 1-9, *s* = 1-6 und *t* = 1 oder 2 ist,
zur Verwendung bei der Behandlung oder Vorbeugung von Infektionen und Entzündungen der Haut oder der Schleimhäute, die durch HSV 1 und HSV 2 verursacht werden, wobei die Behandlung oder Vorbeugung Mischen der Zusammensetzung (I) und (II) und Verabreichen des Gemisches der Zusammensetzungen an ein Subjekt aufweist.

4. Zusammensetzung (III), erhältlich durch Mischen der Zusammensetzungen (I) und (II), wobei die Zusammensetzung (I) erhältlich ist mittels:
Herstellen eines Gemisches aus:
(a) Glycerol bzw. Glycerin;
(b) einem oder mehreren Cellulose-Verdickungsmitteln, vorzugsweise Carboxymethylcellulose; und
(c) optional einem oder mehreren Gummi bzw. Gummisorten, ausgewählt aus Xanthangummi, Carrageenan, Chiclegummi (aus Sapodilla), Guargummi, Gummi arabicum, Akaziengummi, Natriumalginat und Tragantgummi,
und mittels Zugeben von Folgendem zu diesem Gemisch:
(d) ein oder mehrerer Kationen C^{*t*+};
(e) ein oder mehrerer Anionen abgeleitet von Halogenoxiden der allgemeinen Formel [XO*ᵥ*]⁻; und
(f) Wasser,
und zwar mit der Maßgabe, dass kein Sorbitol zugesetzt wird, wobei:
C ein Metall der Gruppe 1 oder 2 des Periodensystems ist,
*t =* 1 oder 2 ist, X ein Halogenatom ist und *v* = 1-4 ist,
wobei die Zusammensetzung (II) erhältlich ist mittels:
Herstellen eines Gemisches aus:
(g) Glycerin; und
(h) einem oder mehreren Cellulose-Verdickungsmitteln, vorzugsweise Carboxymethylcellulose; und
(i) einem oder mehreren Gummi bzw. Gummisorten, ausgewählt aus Xanthangummi, Carrageen, Chiclegummi (aus Sapodilla), Guargummi, Gummi arabicum, Akaziengummi, Natriumalginat und Tragantgummi, vorzugsweise Xanthangummi,
und mittels Zugeben von Folgendem zu diesem Gemisch:
(j) eines oder mehrerer Sauerstoffdonatoren, wobei ein Sauerstoffdonator ein Kation C^{*t*+} und ein Anion gemäß der allgemeinen Formel [Z*_{y}*O*ₓ*]^{*s*-}aufweist; und
(k) Wasser,
und zwar mit der Maßgabe, dass kein Sorbitol zugesetzt wird, wobei:
C ein Metall der Gruppe 1 oder 2 des Periodensystems ist, Z aus den Elementen Bor, Mangan, Kohlenstoff, Schwefel, Phosphor, Calcium und Magnesium ausgewählt ist, und wobei O das Element Sauerstoff ist, *y* = 0-4, *x* = 1-9, *s* = 1-6 und *t* = 1 oder 2 ist,
zur Verwendung bei der Behandlung oder Vorbeugung von Infektionen und Entzündungen der Haut oder der Schleimhäute, die durch HSV 1 und HSV 2 verursacht werden, wobei die Behandlung oder Vorbeugung Verabreichen des Gemisches der Zusammensetzung (III) an ein Subjekt aufweist.

5. Bausatz zur Verwendung nach Anspruch 3 oder Zusammensetzung (III) zur Verwendung nach Anspruch 4, wobei durch HSV 1 und HSV 2 verursachte Infektionen und Entzündungen der Haut oder Schleimhäute Primärinfektionen der Haut oder Schleimhäute sind, auf die sekundäre bakterielle oder mykotische Infektionen folgen.

6. Bausatz zur Verwendung nach Anspruch 3 oder Zusammensetzung (III) zur Verwendung nach Anspruch 4, wobei Infektionen und Entzündungen der Haut oder der Schleimhäute, die mit HSV 1 und HSV 2 zusammenhängen, aus der Gruppe bestehend aus Ekzemen und Psoriasis ausgewählt sind.

7. Bausatz zur Verwendung nach Anspruch 3 oder Zusammensetzung (III) zur Verwendung nach Anspruch 4, wobei die Infektion und Entzündung der Haut oder der Schleimhäute verursacht durch Viren, die durch denselben Mechanismus wie HSV 1 und HSV 2 wirken, ein Mundgeschwür ist.

8. Bausatz oder Zusammensetzung (III) zur Verwendung nach einem der Ansprüche 1 bis 7, wobei das eine oder die mehreren von Halogenoxiden abgeleiteten Anionen gemäß der allgemeinen Formel [XO*ᵥ*]⁻ in Zusammensetzung (I) ausgewählt sind aus der Gruppe bestehend aus Perchlorat, Chlorat, Chlorit, Hypochlorit, Perioidat, Jodat, Jodid, Hypojodid, Perbromat, Bromat, Bromit und Hypobromit, wobei:
(i) das eine oder die mehreren von Halogenoxiden abgeleiteten Anionen gemäß der allgemeinen Formel [XO*ᵥ*]⁻ in Zusammensetzung (I) vorzugsweise Hypochlorit aufweisen; oder
(ii) die Zusammensetzung (I) vorzugsweise ein Kation C^{*t*+} und ein von Halogenoxiden abgeleitetes Anion gemäß der allgemeinen Formel [XO*ᵥ*]⁻ aufweist, wobei das Kation und das Anion der Zusammensetzung als wässrige Lösung von Natriumhypochlorit zugesetzt werden.

9. Bausatz oder Zusammensetzung (III) zur Verwendung nach einem der Ansprüche 1 bis 8, wobei das Anion gemäß der allgemeinen Formel [Z*_{y}*O*ₓ*]^{*s*-}in dem einen oder den mehreren Sauerstoffdonatoren der Zusammensetzung (II) ausgewählt ist aus der Gruppe bestehend aus Perborat, Metaborat, Orthoborat, Hypoborat, Pyroborat, Tetraborat, Persulfat, Peroxid, Permanganat, Percarbonat, Perphosphat und deren Hydraten.

10. Bausatz oder Zusammensetzung (III) zur Verwendung nach einem der Ansprüche 1 bis 9, wobei Z Bor ist und das Anion gemäß der allgemeinen Formel [Z*_{y}*O*ₓ*]^{*s*-} in dem einen oder den mehreren Sauerstoffdonatoren der Zusammensetzung (II) ausgewählt ist aus der Gruppe bestehend aus Perborat, Metaborat, Orthoborat, Hypoborat, Pyroborat und Tetraborat und deren Hydraten, vorzugsweise Perborat.

11. Bausatz oder Zusammensetzung (III) zur Verwendung nach einem der Ansprüche 1 bis 9, wobei Z Kohlenstoff ist und das Anion gemäß der allgemeinen Formel [Z*_{y}*O*ₓ*]^{*s*-} in dem einen oder mehreren Sauerstoffdonatoren der Zusammensetzung (II) Percarbonat ist.

12. Bausatz oder Zusammensetzung (III) zur Verwendung nach einem der Ansprüche 1 bis 11, wobei das Gemisch aus den Zusammensetzungen (I) und (II) oder die Zusammensetzung (III) mit einem oder mehreren pharmazeutisch verträglichen Trägern und/oder pharmazeutisch verträglichen Additiven kombiniert ist bzw. wird, bevor das Gemisch aus den Zusammensetzungen (I) und (II) oder die Zusammensetzung (III) dem bedürftigen Subjekt verabreicht wird.

13. Bausatz oder Zusammensetzung (III) zur Verwendung nach Anspruch 12, wobei der eine oder die mehreren pharmazeutisch verträglichen Träger und/oder die pharmazeutisch verträglichen Additive aus der Gruppe ausgewählt sind bestehend aus Bindemitteln, Verdickungsmitteln, Stabilisatoren, Geschmacksstoffen, Duftstoffen, Aromen, Vitaminen und Co-Enzymen,
wobei vorzugsweise:
(i) das Bindemittel aus der Gruppe ausgewählt ist, die aus Glycerin und Gummi besteht; oder
(ii) das Bindemittel aus der Gruppe ausgewählt ist, die aus Glycerin und Gummi besteht und das Verdickungsmittel Carboxymethylcellulose ist; oder
(iii) das Verdickungsmittel Carboxymethylcellulose ist.

14. Bausatz oder Zusammensetzung (III) zur Verwendung nach einem der Ansprüche 1 bis 13, wobei das Gemisch aus den Zusammensetzungen (I) und (II) oder wobei die Zusammensetzung (III), optional in Kombination mit einem oder mehreren pharmazeutisch verträglichen Trägern und/oder pharmazeutisch verträglichen Zusatzstoffen, die Form einer Flüssigkeit oder eines Feststoffs, insbesondere eines Pulvers, einer wässrigen Lösung, einer wässrigen Dispersion, eines Gels, einer Mousse, eines Sprays, einer Seife, eines Shampoos, einer Paste, eines Tascheninjektionsgels, einer Salbe oder eines Balsams einnimmt.

15. Bausatz oder Zusammensetzung (III) zur Verwendung nach einem der Ansprüche 1 bis 14, wobei der eine oder die mehreren Gummi bzw. Gummisorten ausgewählt aus Xanthangummi, Carrageen, Chiclegummi (aus Sapodilla), Guargummi, Gummi arabicum, Akaziengummi, Natriumalginat und Tragantgummi, vorzugsweise Xanthangummi, obligatorische Bestandteile der Zusammensetzung (I) und (II) ist bzw. sind.

## Revendications

1. Kit de pièces comprenant des compositions (I) et (II) dans des récipients séparés,
dans lequel la composition (I) peut être obtenue en :
préparant un mélange de :
(a) glycérol ;
(b) un ou plusieurs épaississants cellulosiques, de préférence de la carboxyméthylcellulose ; et
(c) facultativement une ou plusieurs gommes choisies parmi de la gomme xanthane, carraghénine, gomme chicle (de sapotille), gomme de guar, gomme arabique, gomme d'acacia, alginate de sodium et gomme adragante,
et en ajoutant audit mélange :
(d) un ou plusieurs cations C^{*t*+} ;
(e) un ou plusieurs anions dérivés d'halogénoxydes selon la formule générale [XO*ᵥ*]⁻ ; et
(f) de l'eau,
à condition de ne pas ajouter de sorbitol,
dans lequel C est un métal du groupe 1 ou 2 de la classification périodique, *t* = 1 ou 2, X est un atome d'halogène et *v* = 1 à 4,
dans lequel la composition (II) peut être obtenue en :
préparant un mélange de :
(g) glycérol ; et
(h) un ou plusieurs épaississants cellulosiques, de préférence de la carboxyméthylcellulose ; et
(i) une ou plusieurs gommes choisies parmi de la gomme xanthane, carraghénine, gomme chicle (de sapotille), gomme de guar, gomme arabique, gomme d'acacia, alginate de sodium et gomme adragante, de préférence de la gomme xanthane,
et en ajoutant audit mélange :
(j) un ou plusieurs donneurs d'oxygène, dans lequel un donneur d'oxygène comprend un cation C^{*t*+} et un anion selon la formule générale [Z*_{y}*O*ₓ*]^{*s*-} ; et
(k) de l'eau,
à condition de ne pas ajouter de sorbitol,
dans lequel C est un métal du groupe 1 ou 2 de la classification périodique, Z est choisi parmi les éléments bore, manganèse, carbone, soufre, phosphore, calcium et magnésium, et dans lequel O est l'élément oxygène, *y* = 0 à 4, *x* = 1 à 9, *s* = 1 à 6 et *t* = 1 ou 2,
pour une utilisation dans le traitement ou la prévention d'infections et d'inflammations bactériennes ou fongiques dans la bouche, ledit traitement ou ladite prévention comprenant le mélange de compositions (I) et (II) et l'administration du mélange de compositions à un sujet, dans lequel les infections et inflammations bactériennes ou fongiques dans la bouche sont de préférence choisies dans le groupe constitué de gingivite, parodontite et péri-implantite.

2. Composition (III) pouvant être obtenue en mélangeant des compositions (I) et (II),
dans laquelle la composition (I) peut être obtenue en :
préparant un mélange de :
(a) glycérol ;
(b) un ou plusieurs épaississants cellulosiques, de préférence de la carboxyméthylcellulose ; et
(c) facultativement une ou plusieurs gommes choisies parmi de la gomme xanthane, carraghénine, gomme chicle (de sapotille), gomme de guar, gomme arabique, gomme d'acacia, alginate de sodium et gomme adragante,
et en ajoutant audit mélange :
(d) un ou plusieurs cations C^{*t*+} ;
(e) un ou plusieurs anions dérivés d'halogénoxydes selon la formule générale [XO*ᵥ*]⁻ ; et
(f) de l'eau,
à condition de ne pas ajouter de sorbitol,
dans laquelle C est un métal du groupe 1 ou 2 de la classification périodique, *t* = 1 ou 2, X est un atome d'halogène et *v* = 1 à 4,
dans laquelle la composition (II) peut être obtenue en :
préparant un mélange de :
(g) glycérol ; et
(h) un ou plusieurs épaississants cellulosiques, de préférence de la carboxyméthylcellulose ; et
(i) une ou plusieurs gommes choisies parmi de la gomme xanthane, carraghénine, gomme chicle (de sapotille), gomme de guar, gomme arabique, gomme d'acacia, alginate de sodium et gomme adragante, de préférence de la gomme xanthane,
et en ajoutant audit mélange :
(j) un ou plusieurs donneurs d'oxygène, dans laquelle un donneur d'oxygène comprend un cation C^{*t*+} et un anion selon la formule générale [Z*_{y}*O*ₓ*]^{*s*-} ; et
(k) de l'eau,
à condition de ne pas ajouter de sorbitol,
dans laquelle C est un métal du groupe 1 ou 2 de la classification périodique, Z est choisi parmi les éléments bore, manganèse, carbone, soufre, phosphore, calcium et magnésium, et dans laquelle O est l'élément oxygène, *y* = 0 à 4, *x* = 1 à 9, *s* = 1 à 6 et *t* = 1 ou 2,
pour une utilisation dans le traitement ou la prévention d'infections et d'inflammations bactériennes ou fongiques dans la bouche, ledit traitement ou ladite prévention comprenant l'administration de la composition (III) à un sujet, dans laquelle les infections et inflammations bactériennes ou fongiques dans la bouche sont de préférence choisies dans le groupe constitué de gingivite, parodontite et péri-implantite.

3. Kit de pièces comprenant des compositions (I) et (II) dans des récipients séparés,
dans lequel la composition (I) peut être obtenue en :
préparant un mélange de :
(a) glycérol ;
(b) un ou plusieurs épaississants cellulosiques, de préférence de la carboxyméthylcellulose ; et
(c) facultativement une ou plusieurs gommes choisies parmi de la gomme xanthane, carraghénine, gomme chicle (de sapotille), gomme de guar, gomme arabique, gomme d'acacia, alginate de sodium et gomme adragante,
et en ajoutant audit mélange :
(d) un ou plusieurs cations C^{*t*+} ;
(e) un ou plusieurs anions dérivés d'halogénoxydes selon la formule générale [XO*ᵥ*]⁻ ; et
(f) de l'eau,
à condition de ne pas ajouter de sorbitol,
dans lequel C est un métal du groupe 1 ou 2 de la classification périodique, *t* = 1 ou 2, X est un atome d'halogène et *v* = 1 à 4,
dans lequel la composition (II) peut être obtenue en :
préparant un mélange de :
(g) glycérol ; et
(h) un ou plusieurs épaississants cellulosiques, de préférence de la carboxyméthylcellulose ; et
(i) une ou plusieurs gommes choisies parmi de la gomme xanthane, carraghénine, gomme chicle (de sapotille), gomme de guar, gomme arabique, gomme d'acacia, alginate de sodium et gomme adragante, de préférence de la gomme xanthane,
et en ajoutant audit mélange :
(j) un ou plusieurs donneurs d'oxygène, dans lequel un donneur d'oxygène comprend un cation C^{*t*+} et un anion selon la formule générale [Z*_{y}*O*_{z}*]^{*s*-} ; et
(k) de l'eau,
à condition de ne pas ajouter de sorbitol,
dans lequel C est un métal du groupe 1 ou 2 de la classification périodique, Z est choisi parmi les éléments bore, manganèse, carbone, soufre, phosphore, calcium et magnésium, et dans lequel O est l'élément oxygène, *y* = 0 à 4, *x* = 1 à 9, *s* = 1 à 6 et *t* = 1 ou 2,
pour une utilisation dans le traitement ou la prévention d'infections et d'inflammations de la peau ou de membranes muqueuses causées par HSV 1 et HSV 2, ledit traitement ou ladite prévention comprenant le mélange des compositions (I) et (II) et l'administration du mélange de compositions à un sujet.

4. Composition (III) pouvant être obtenue en mélangeant des compositions (I) et (II),
dans laquelle la composition (I) peut être obtenue en :
préparant un mélange de :
(a) glycérol ;
(b) un ou plusieurs épaississants cellulosiques, de préférence de la carboxyméthylcellulose ; et
(c) facultativement une ou plusieurs gommes choisies parmi de la gomme xanthane, carraghénine, gomme chicle (de sapotille), gomme de guar, gomme arabique, gomme d'acacia, alginate de sodium et gomme adragante,
et en ajoutant audit mélange :
(d) un ou plusieurs cations C^{*t*+} ;
(e) un ou plusieurs anions dérivés d'halogénoxydes selon la formule générale [XO*ᵥ*]⁻ ; et
(f) de l'eau,
à condition de ne pas ajouter de sorbitol,
dans laquelle C est un métal du groupe 1 ou 2 de la classification périodique, *t* = 1 ou 2, X est un atome d'halogène et *v* = 1 à 4,
dans laquelle la composition (II) peut être obtenue en :
préparant un mélange de :
(g) glycérol ; et
(h) un ou plusieurs épaississants cellulosiques, de préférence de la carboxyméthylcellulose ; et
(i) une ou plusieurs gommes choisies parmi de la gomme xanthane, carraghénine, gomme chicle (de sapotille), gomme de guar, gomme arabique, gomme d'acacia, alginate de sodium et gomme adragante, de préférence de la gomme xanthane,
et en ajoutant audit mélange :
(j) un ou plusieurs donneurs d'oxygène, dans laquelle un donneur d'oxygène comprend un cation C^{*t*+} et un anion selon la formule générale [*Z_{y}*O*ₓ*]^{*s*-} ; et
(k) de l'eau,
à condition de ne pas ajouter de sorbitol,
dans laquelle C est un métal du groupe 1 ou 2 de la classification périodique, Z est choisi parmi les éléments bore, manganèse, carbone, soufre, phosphore, calcium et magnésium, et dans laquelle O est l'élément oxygène, *y* = 0 à 4, *x* = 1 à 9, *s* = 1 à 6 et *t* = 1 ou 2,
pour une utilisation dans le traitement ou la prévention d'infections et d'inflammations de la peau ou de membranes muqueuses causées par HSV 1 et HSV 2, ledit traitement ou ladite prévention comprenant l'administration de la composition (III) à un sujet.

5. Kit de pièces à utiliser selon la revendication 3 ou composition (III) à utiliser selon la revendication 4, dans lesquels les infections et inflammations de la peau ou de membranes muqueuses causées par HSV 1 et HSV 2 sont des infections primaires de la peau ou de membranes muqueuses qui sont suivies d'infections bactériennes ou fongiques secondaires.

6. Kit de pièces à utiliser selon la revendication 3 ou composition (III) à utiliser selon la revendication 4, dans lesquels les infections et inflammations de la peau ou de membranes muqueuses liées à HSV 1 et HSV 2 sont choisies dans le groupe constitué d'eczéma et psoriasis.

7. Kit de pièces à utiliser selon la revendication 3 ou composition (III) à utiliser selon la revendication 4, dans lesquels l'infection et l'inflammation de la peau ou de membranes muqueuses causées par des virus agissant par le même mécanisme que HSV 1 et HSV 2 sont un ulcère de la bouche.

8. Kit de pièces ou composition (III) à utiliser selon l'une quelconque des revendications 1 à 7, dans lesquels les un ou plusieurs anions dérivés d'halogénoxydes selon la formule générale [XO*ᵥ*]⁻ dans la composition (I) sont choisis dans le groupe constitué de perchlorate, chlorate, chlorite, hypochlorite, périodate, iodate, iodite, hypoiodite, perbromate, bromate, bromite et hypobromite, dans lesquels :
(i) les un ou plusieurs anions dérivés d'halogénoxydes selon la formule générale [XO*ᵥ*]⁻ dans la composition (I) comprennent de préférence de l'hypochlorite ; ou
(ii) la composition (I) comprend de préférence un cation C^{*t*+} et un anion dérivé d'halogénoxydes selon la formule générale [XO*ᵥ*]⁻, lesdits cation et anion étant ajoutés à la composition sous forme d'une solution aqueuse d'hypochlorite de sodium.

9. Kit de pièces ou composition (III) à utiliser selon l'une quelconque des revendications 1 à 8, dans lesquels l'anion selon la formule générale [Z*_{y}*O*ₓ*]^{*s*-} dans les un ou plusieurs donneurs d'oxygène de la composition (II) est choisi dans le groupe constitué de perborate, métaborate, orthoborate, hypoborate, pyroborate, tétraborate, persulfate, peroxyde, permanganate, percarbonate, perphosphate, et leurs hydrates.

10. Kit de pièces ou composition (III) à utiliser selon l'une quelconque des revendications 1 à 9, dans lesquels Z est du bore et l'anion selon la formule générale [Z*_{y}*O*ₓ*]^{*s*-} dans les un ou plusieurs donneurs d'oxygène de la composition (II) est choisi dans le groupe constitué de perborate, métaborate, orthoborate, hypoborate, pyroborate, et tétraborate, et leurs hydrates, de préférence du perborate.

11. Kit de pièces ou composition (III) à utiliser selon l'une quelconque des revendications 1 à 9, dans lequel Z est du carbone et l'anion selon la formule générale [Z*_{y}*O*ₓ*]^{*s*-} dans les un ou plusieurs donneurs d'oxygène de la composition (II) est du percarbonate.

12. Kit de pièces ou composition (III) à utiliser selon l'une quelconque des revendications 1 à 11, dans lesquels le mélange des compositions (I) et (II) ou la composition (III) est combiné à un ou plusieurs supports pharmaceutiquement acceptables et/ou additifs pharmaceutiquement acceptables avant l'administration du mélange de compositions (I) et (II) ou de la composition (III) au sujet qui en a besoin.

13. Kit de pièces ou composition (III) à utiliser selon la revendication 12, dans lesquels les un ou plusieurs supports pharmaceutiquement acceptables et/ou additifs pharmaceutiquement acceptables sont choisis dans le groupe constitué de liants, épaississants, stabilisants, aromatisants, parfums, arômes, vitamines et co-enzymes,
dans lesquels de préférence :
(i) le liant est choisi dans le groupe constitué de glycérol et gommes ; ou
(ii) le liant est choisi dans le groupe constitué de glycérol et gommes et l'épaississant est de la carboxyméthylcellulose ; ou
(iii) l'épaississant est de la carboxyméthylcellulose.

14. Kit de pièces ou composition (III) à utiliser selon l'une quelconque des revendications 1 à 13, dans lesquels le mélange de compositions (I) et (II) ou dans lesquels la composition (III), facultativement combiné à un ou plusieurs supports pharmaceutiquement acceptables et/ou additifs pharmaceutiquement acceptables, se présente sous la forme d'un liquide ou d'un solide, en particulier une poudre, une solution aqueuse, une dispersion aqueuse, un gel, une mousse, un spray, un savon, un shampoing, une pâte, un gel d'injection dans les poches, un onguent ou une pommade.

15. Kit de pièces ou composition (III) à utiliser selon l'une quelconque des revendications 1 à 14, dans lesquels les une ou plusieurs gommes choisies parmi de la gomme xanthane, carraghénine, gomme chicle (de sapotille), gomme de guar, gomme arabique, gomme d'acacia, alginate de sodium et gomme adragante, de préférence la gomme xanthane, sont des ingrédients obligatoires dans les compositions (I) et (II).
